# EUROPEAN PATENT SPECIFICATION

(11) **EP 0 893 075 B1**
(45) Date of publication and mention of the grant of the patent: **15.05.2002**
(21) Application number: 98305929.6
(22) Date of filing: 24.07.1998
(51) Int. Cl.: A44B 18/00, A61F 13/58

(54) **Surface fastener**
Flächenhaftverschluss
Fermeture à éléments d'accrochage

(30) Priority: 25.07.1997 GB 9715836
(43) Date of publication of application: 27.01.1999
(73) Proprietor: YKK CORPORATION, Chiyoda-ku, Tokyo (JP)
(72) Inventor: Kato, Hisanori, 60488 Frankfurt (DE)
(74) Representative: Luckhurst, Anthony Henry William

(56) References cited:
- EP-A- 0 709 038
- US-A- 3 176 364
- US-A- 5 636 414

## Description

### BACKGROUND OF THE INVENTION

### 1. Field of the Invention

The present invention relates to a surface fastener.

### 2. Description of the Related Art

WO96/25905 (which corresponds to US 5 636 414 A) describes a hook type surface fastener in which the co-operating surface fastener parts have, in addition to the mechanical hook and loop fastening, a coating of cohesive material on each part. The cohesive coatings are said to operate to enhance the engagement between the surface fastener parts to provide increased shear and peel resistance. Cohesive materials are materials which will normally bond only with another cohesive material, forming a strong bond to a like cohesive material. The materials may be water based, hot melt materials, cross-linked polymers, and tackless cohesive materials are also available.

In WO96/25905, a hook shaped fastener part has a coating of cohesive material applied to the outer surface of the hooks, the outer surface being to face the co-operating companion fastener part as the fastener parts are brought together. The cohesive material may also be applied to the surface of the base material from which the hooks project.

In this type of surface fastener, engaging and peeling are repeated during use. And in case of continuously manufacturing the hook shaped fastener part, a long strip of molded product is wound in a roll. In order to avoid loosening of the roll and to facilitate subsequent attaching to articles, the cohesive may often be applied on a rear surface of the hook shaped fastener part.

In case that the adhesive is applied directly onto the outer surface of the hooks to form the coating, the hook shaped fastener part adheres the companion fastener part at the time of engaging and peeling of the surface fastener or unwinding of the wound hook shaped fastener part, so that the adhesive moves to the companion fastener part or removed off the outer surface of the hooks of the fastener part.

This invention is developed to solve the foregoing problems, and its object is to provide a surface fastener in which though the hook shaped fastener parts are integrally molded of synthetic resin and have shape with which a great engaging strength is hard to realize mechanically, a desired shearing resistance and peeling resistance can be obtained and its durability can also be secured.

### SUMMARY OF THE INVENTION

The present invention provides a surface fastener comprising a substrate and engaging elements extending from the substrate, and the engaging elements have a recess and a cohesive or adhesive material is embedded in the recess.

Even in the case that a desired shearing resistance and peeling resistance are hard to be secured due to the shape of the engaging elements, partly since such resistance can be secured due to the cohesive material on a part of the engaging elements and partly since the cohesive is embedded in the recess formed in the engaging elements, the cohesive does not get removed by contacting other parts, so that repetitive use and unwinding hardly deteriorate the shearing resistance and peeling resistance to improve the durability.

It is preferable that the engaging elements are molded hooks, and the recess is provided in a surface of the hook which surface faces away from the substrate. Further preferably, the hooks are crook shaped, and the recess is formed in the outer surface of the hook at the top of the crook. Also preferably, the recess is a groove formed in the outer surface of the hook.

Alternatively, the hook may comprise a body portion extending upwardly at a first angle to the substrate, and a nose portion extending laterally from the body portion, and away from the substrate at a second angle to the substrate which is less than the first angle, and a flattened region is formed on a surface of the nose portion and faces away from the substrate. In such case, it is preferable that the recess is formed in the region of the flattened region.

By embedding the cohesive or adhesive material in the recess, the risk of the material being worn away before use, or removed off, or contaminating other articles is reduced.

### BRIEF DESCRIPTION OF THE DRAWINGS

FIG. 1 is a fragmentary perspective view of a first embodiment of a surface fastener according to the invention;
FIG. 2 is a fragmentary perspective view showing the configuration of the surface fastener of FIG. 1 before a cohesive material is embedded;
FIG. 3 is a plan view of a hook according to a second embodiment of a surface fastener of the invention;
FIG. 4 is a fragmentary side view showing a hook of the surface fastener in which a flattened surface nor a groove is formed;
FIG. 5 is a fragmentary side view showing the hook shape of FIG. 4 after the flattened surface is formed; and
FIG. 6 is a fragmentary side view showing the shape of the surface fastener in which the groove is formed before the flattened surface is formed.

### PREFERRED EMBODIMENTS OF THE INVENTION

The invention will be further described by way of example, with reference to the accompanying drawings.

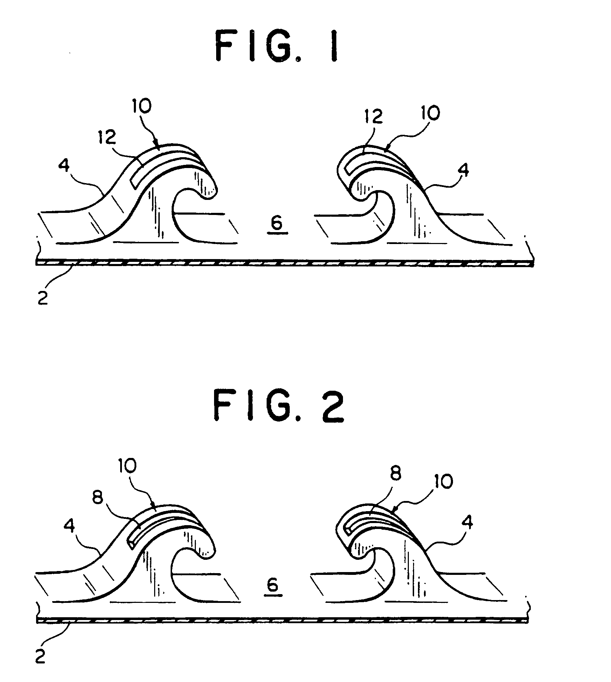

FIG. 1 is a perspective view of a first embodiment of a surface fastener according to the invention, and FIG. 2 shows the surface fastener before a cohesive material is embedded.

The surface fastener of the first embodiment is a general hook type surface fastener having a shape shown in FIG. 2. The construction and manufacture of foregoing integrally molded hook fasteners is well known and described, for example in EP-A-0 267 193 and EP-A-0 464 753. For the purpose of illustration only two hooks are shown. It will be appreciated by those in the art that the fastener comprises a multiplicity of hooks having illustrated shape, which are typically arranged in rows and may face in the same or different directions.

The hooks shown in FIG. 2 differ from the prior art hooks in the provision of a groove in the outer surface of the hook, to receive a layer of cohesive or adhesive material as shown in FIG. 1.

In more detail, the molded hook type surface fastener shown in FIG. 2 comprises hooks 4 integrally molded and projecting from an upper surface 6 of a strip of substrate 2. A groove 8 serving as a recess in the invention is formed on the outer surface 10 of the hooks 4. An outer surface position of the groove 8 is located at the top of the hook 4 so that the groove 8 extends over the top thereof. The groove 8 is formed in the outer surface 10 of the hook 4 during molding.

Referring to FIG. 1, the grooves 8 of the hooks 4 are filled with a cohesive or adhesive material 12. In use the cohesive material 12 is arranged to mate with cohesive material provided on a complementary substrate or engaging elements to be engaged by the surface fastener. When the groove 8 is filled with adhesive material, the substrate need not have a complementary material coating. Thus the adhesive material may be a pressure sensitive adhesive, and a peelable adhesive could be used for temporary location of the fastener on a substrate at the time of winding or the like.

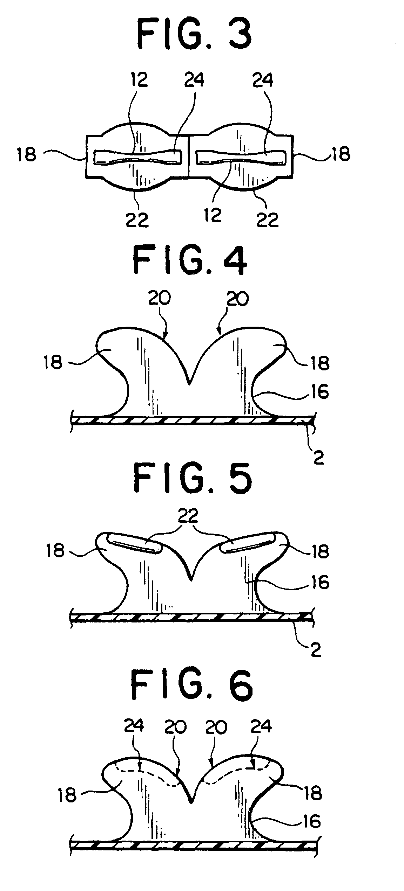

FIG. 3 shows another type of molded hook type surface fastener, forming another embodiment of the invention, based on a prior art hook shape illustrated in FIGS. 4 and 5. The prior art surface fastener is formed by firstly molding a hook shape of FIG. 4 in which a body portion 16 extends upwardly perpendicular to the substrate 2 and then a nose portion 18 extends laterally from the body portion 16 at a shallower angle. In the embodiment shown, two nose portions 18 extends on the body portion 16 directing opposite each other.

The upper surfaces 20 of the nose portions 18 are plastic-deformed to flatten them, forming lobes or ears 22 which project to each side of the nose portion 18, as shown in FIG. 5. The upper surfaces 20 are formed by passing the nose portion 18 under a heated roller.

FIG. 6 shows a side view of the preform for the embodiment of FIG. 3, molded with a groove 24 in its upper surface 20. The upper surface 20 is then flattened by a forming process of the prior art shown in FIGS. 4 and 5, and the groove 24 then filled with cohesive or adhesive materials 12, as shown in plan view in FIG. 3.

These illustrated embodiments show typical examples of the invention, and it shall be easily understood that the invention should not be limited to these examples and various modifications are possible within the scope of the invention.

## Claims

1. A surface fastener comprising a substrate (2) and engaging elements extending from the substrate (2), said surface fastener being **characterized by** that the engaging elements have a recess and a cohesive or adhesive material (12) is embedded in the recess.

2. A surface fastener as claimed in claim 1, wherein the engaging elements are molded hooks (4), and the recess is provided in a surface of the hook (4) which surface faces away from the substrate.

3. A surface fastener as claimed in claim 2, wherein the hooks (4) are crook shaped, and the recess is formed in the outer surface of the hook (4) at the top of the crook.

4. A surface fastener as claimed in claim 3, wherein the recess is a groove (8) formed in the outer surface of the hook (4).

5. A surface fastener as claimed in claim 2, wherein the hook (4) comprises a body portion (16) extending upwardly at a first angle to the substrate (2), and a nose portion (18) extending laterally from the body portion (16), and away from the substrate (2) at a second angle to the substrate (2) which is less than the first angle, and a flattened region is formed on a surface of the nose portion (18) and faces away from the substrate (2).

6. A surface fastener as claimed in claim 5, wherein the recess is formed in the region of the flattened region.

## Patentansprüche

1. Flächenhaftverschluss, welcher ein Trägermaterial (2) und Eingreifelemente aufweist, die sich vom Trägermaterial (2) aus erstrecken, wobei der Flächenhaftverschluss **dadurch gekennzeichnet ist, dass** die Eingreifelemente eine Vertiefung aufweisen und ein Kohäsiv- oder Adhäsivmaterial (12) in die Vertiefung eingebettet ist.

2. Flächenhaftverschluss nach Anspruch 1, bei welchem die Eingreifelemente formgegossene Haken (4) sind und die Vertiefung in einer Fläche des Hakens (4) vorgesehen ist, welche in Richtung vom Trägermaterial weg weist.

3. Flächenhaftverschluss nach Anspruch 2, bei welchem die Haken (4) bogenförmig gekrümmt sind und die Vertiefung in der Außenfläche des Hakens (4) an der Oberseite des Hakens ausgebildet ist.

4. Flächenhaftverschluss nach Anspruch 3, bei welchem die Vertiefung eine Nut (8) ist, die in der Außenfläche des Hakens (4) ausgebildet ist.

5. Flächenhaflverschluss nach Anspruch 2, bei welchem der Haken (4) einen Körperabschnitt (16) aufweist, der sich unter einem ersten Winkel zum Trägermaterial (2) nach oben erstreckt, und einen Nasenabschnitt (18), der sich seitlich vom Körperabschnitt (16) und in Richtung vom Trägermaterial (2) weg erstreckt, und zwar unter einem zweiten Winkel zum Trägermaterial (2), der kleiner ist als der erste Winkel, und ein abgeflachter Bereich auf einer Fläche des Nasenabschnitts (18) ausgebildet ist und in Richtung vom Trägermaterial (2) weg weist.

6. Flächenhaftverschluss nach Anspruch 5, bei welchem die Vertiefung im Bereich des abgeflachten Gebietes ausgebildet ist.

## Revendications

1. Fermeture contact comprenant un substrat (2) et des éléments de mise en prise qui s'étendent à partir du substrat (2), ladite fermeture contact étant **caractérisée en ce que** les éléments de mise en prise comportent un creux et **en ce qu'**un matériau cohésif ou adhésif (12) est incorporé dans ce creux.

2. Fermeture contact selon la revendication 1, dans laquelle les éléments de misé en prise sont des crochets moulés (4) et dans laquelle le creux est ménagé dans une surface du crochet (4), laquelle surface est orientée à l'écart du substrat.

3. Fermeture contact selon la revendication 2, dans laquelle les crochets (4) ont une forme courbe, et dans laquelle le creux est réalisé dans la surface extérieure du crochet (4) au sommet de la partie courbe.

4. Fermeture contact selon la revendication 3, dans laquelle le creux est une rainure (8) réalisée dans la surface extérieure du crochet (4).

5. Fermeture contact selon la revendication 2, dans laquelle le crochet (4) comprend une partie formant corps (16) s'étendant vers le haut en formant un premier angle par rapport au substrat (2), et une partie formant dent (18) s'étendant latéralement à partir de la partie formant corps (16) et s'écartant du substrat (2) en formant un second angle par rapport au substrat (2) qui est inférieur au premier angle, et une zone aplatie est réalisée sur une surface de la partie formant dent (18) et est orientée vers l'extérieur par rapport au substrat (2).

6. Fermeture contact selon la revendication 5, dans laquelle le creux est réalisé dans la région de la zone aplatie.
